# EUROPEAN PATENT APPLICATION

(11) **EP 1 457 179 A1**
(43) Date of publication of application: **15.09.2004**
(21) Application number: 02738816.4
(22) Date of filing: 27.06.2002
(51) Int. Cl.: A61F 9/00

(54) **MEDICAL INSTRUMENT FOR CORNEA OPERATION**

(30) Priority: 30.11.2001 JP 2001367002
(71) Applicant: Kokusai Commerce Inc., Tokyo 102-0083 (JP)
(72) Inventor: TAKAHASHI, Keizo, Miyazaki-shi, Miyazaki 880-0939 (JP)
(74) Representative: de Roquemaurel, Bruno
(86) International application number: PCT/JP2002/006478
(87) International publication number: WO 2003/047480

(57) **Abstract**

Provided here is a medical instrument, which is employed in a surgical operation of a cornea and capable of preparing a corneal epithelium sheet (including a flap and a cap) in a corneal refractive correction surgical operation or a corneal epithelium grafting operation. The medical instrument employed in the corneal surgical operation of the present invention is provided with a thread-like member 5 which has a diameter D of up to 100 µm and is positioned in the boundary between a corneal epithelium 51 and a corneal stroma 55. The thread-like member 5 is provided with: a resiliency which makes it possible for a first contact surface 5a of the thread-like member 5 to deform along a shape of a contact surface of a corneal stroma 55 in response to a driving force F applied to the thread-like member 5 in a direction toward a bottom portion of the corneal stroma 55; and, a strength which makes it possible for a second contact surface 5b of the thread-like member 5 to push up the lowermost bottom surface or contact surface of a corneal epithelium 51 when the thread-like member 5 is driven along the boundary surface 61 between the corneal stroma 55 and the corneal epithelium 51 in response to a thrust f2 applied in a direction along the boundary surface 61.

## Description

### TECHNICAL FIELD:

The present invention relates to a medical instrument for carrying out a surgical operation of cornea, and more particularly to a medical instrument used in a corneal refractive correction surgical operation or a corneal epithelium grafting operation.

### BACKGROUND ART:

A cornea is positioned at a starting point of an optical path in visual function to pass and refract an incident light. Consequently, the cornea is an essential organ to realize a sound visual function.

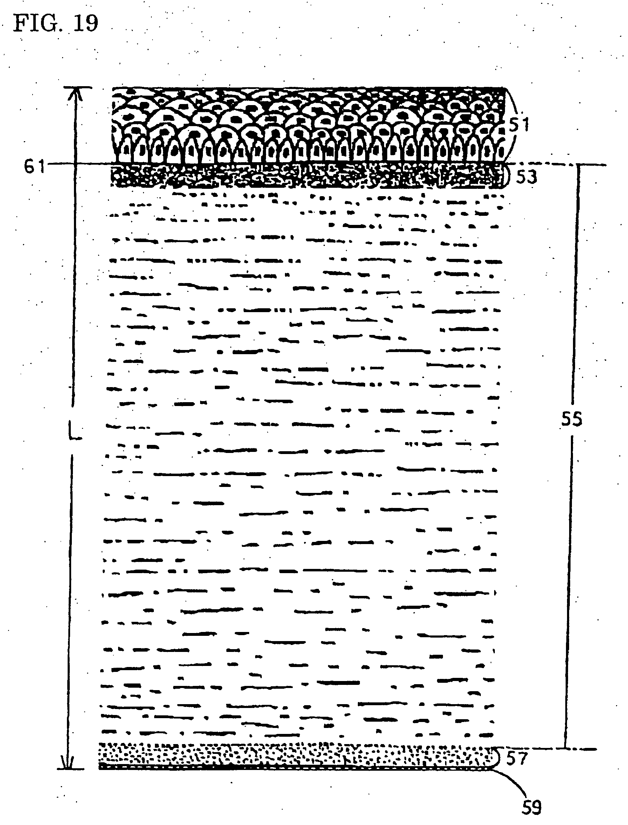

Fig. 19 is a sectional view showing the cornea in construction.

The cornea is constructed of: a corneal epithelium 51 composed of from 6 to 7 layers; Bowman's layer 53; a corneal stroma 55 which forms approximately 80 % of the cornea; Descemet's membrane 57 which is the basal membrane of endothelium cells; and, an endothelium composed of hexagonal cells. The cornea has a thickness of approximately 500 µm (L), in which: the corneal epithelium 51 forms an outer layer; and, the endothelium 59 forms an inner layer. On the other hand, the Bowman's layer 53 forms a part of the corneal stroma 55. Consequently, a boundary surface 61 between the corneal epithelium 51 and the corneal stroma 55 lies between the lowermost bottom portion of the corneal epithelium 51 and the Bowman's layer 53.

Properties of the corneal epithelium 51 are as follows: namely, in a normal state, lively cytodieresis or proliferation of cells is conducted so that the number of cells increases in an area ranging from the lowermost bottom side to a surface of the corneal epithelium 51, whereby old cells in the surface desquamate or come off. In other words, in the corneal epithelium 51, lively replacement of the old cells with new ones is constantly conducted. Due to this, any incised wound is capable of being cured in a short time in the order of days. In curing of the incised wound, the corneal epithelium 51 begins to extend from a peripheral portion of the incised wound, so that the wound is covered by the corneal epithelium 51 which is then increased in thickness to return to its initial sound condition.

On the other hand, properties of the corneal stroma 55 are as follows: namely, innormal condition, the corneal stroma 55 remains substantially unchanged. As for an incised wound appearing in the corneal stroma 55, such wound is slowly cured to require a relatively very long period of time in curing in the order of years. However, a complete recovery is not expected. In many cases, such incised wound having been cured or healed results in dim (cloudy) sight.

In order to correct a visual power of a patient, heretofore employed are eyeglasses or contact lenses. However, in recent years, a corneal refractive correction surgical operation becomes popular.

In general, such corneal refractive correction surgical operation is classified into: a so-called "PRK (photorefractive keratectomy)"; a so-called "LASIK (Laser in situ keratomileusis)"; and, a so-called "LASEK(Laser epithelial keratomileusis)".

The PRK operation is as follows: namely, in this operation, an eyedropper-type anesthesia is conducted, and, then eyelids of the patient are opened by means of an eyelid opener. After that, the corneal epithelium 51 is separated using a so-called "Golf knife" or spatula in order to exclusively remove the corneal epithelium 51. As a result, the corneal stroma 55 is exposed to the outside. The uppermost surface of the thus exposed corneal stroma 55 is subjected to an irradiation of an excimer laser light, so that a surface layer of the corneal stroma 55 is properly ablated to enable the stroma 55 to assume various shapes, which varies the refractive power of the patient's eye globe. As a ftercare of the surgical operation, for example, an orthokeratological-type of contact lenses is worn by the patient.

On the other hand, the LASIK operation is as follows: namely, in this operation, an eyedropper-type anesthesia is conducted, and, then eyelids of the patient are opened by means of an eyelid opener. Under such circumstances, a suction ring is mounted on a face of the patient and used to increase an intraocular pressure of the patient under suction. After that, tonometry or measurement of the intraocular pressure is conducted to determine whether or not the intraocular pressure of the patient reaches a desired value of equal to or more than a pressure of from 65 mmHg to 70 mmHg. Under such. desired intraocular pressure, an incision is conducted by using a so-called microkeratome to realize an interlayer incision of the corneal stroma 55. Through such incision, a hinged corneal sheet is prepared. This hinged corneal sheet has its part still connected with the remaining portion of the corneal stroma 55. Incidentally, the corneal sheet including the corneal epithelium 51 has a thickness of approximately 150 µm. Since the corneal epithelium has a thickness of approximately 50 µm, the microkeratome incises the corneal stroma 55 at a depth of approximately 100 µm. The corneal stroma 55 has its incised and exposed surface irradiated by the excimer laser light, so that the exposed surface of the corneal stroma 55 is ablated to change the refractive power of the eye globe. After that, the corneal sheet is replaced or returned to its initial position.

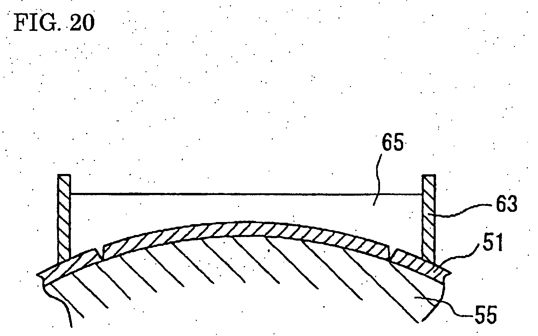

As for the LASEK operation, this operation is as follows: namely, in this operation, an eyedropper-type anesthesia is conducted, and, then eyelids of the patient are opened by means of an eyelid opener. Under such circumstances, marking on the corneal epithelium 51 is made by using a so-called "epithelium trepan" in order to prepare the hinged sheet of the corneal epithelium 51, wherein the epithelium trepan (which partially lacks its blade in order to prepare the hinged portion of the sheet) is used to draw a boundary line for preparing the hinged corneal epithelium sheet. Then, as shown in Fig. 20, alcohol 65 is poured into an alcohol receiver 63, so that impregnation with alcohol is conducted to have the corneal epithelium sheet be free or separate from the corneal stroma 55, wherein the corneal sheet is constructed of the corneal epithelium. After that, surface wash is conducted with the use of a physiological salt solution or saline. Then, separation of the corneal epithelium 51 begins to start. Such separation is a delicate operation requiring a long time of approximately 10 minutes due to fragility of the corneal epithelium itself. After completion of such separation operation, removal of the residues of the corneal epithelium 51 is conducted. Then, the excimer laser light is irradiated onto the uppermost exposed surface of the corneal stroma 55, so that the surface layer of the corneal stroma 55 is ablated to vary in shape, whereby the corneal refractive power is changed. After that, in order to stretch the corneal epithelium, water is supplied so that the corneal epithelium is stretched and replaced or returned to its initial position. This replacement or return operation is also a delicate operation as well as the above-mentioned separation operation. Incidentally, as for this corneal epithelium sheet, since epithelium cells of this sheet is dehydrated and immobilized through the medication, all the cells of the epithelium are destroyed. Due to this, as an aftercare treatment until the corneal epithelium 51 is regenerated, for example, the orthokeratological contact. lenses are worn by the patient.

Further, in the field of the corneal surgical operation, there is also a corneal grafting operation. This corneal grafting operation is classified into: an allograft operation; a grafting operation of a superficial layer of cornea; and, a grafting operation of a deep superficial layer of cornea. Of these operations, the allograft operation is a tissue transplant to the patient or recipient from a donor of another individual, wherein: all the layers of the cornea of the another individual are gouged out by a depth of from approximately 200 µm to approximately 300 µm measured from the surface of the cornea; and, the thus gouged-out piece of the cornea is then sewed to the patient' cornea. On the other hand, in the grafting operation of the deep superficial layer of: the cornea of another person is gouged out in a condition in which all the layers or only the endothelium are or is separated. Under such circumstances, thus separated piece of the cornea of the another person is sewed to the cornea of the patient who has both his endothelium and Descmet's membrane remained in this eye globe.

On the other hand, in recent years, there appears another surgical technique. This another technique, though it is not a technique for culturing the sheet constructed of only the corneal epithelium 51, cultures the corneal epithelium 51 in a condition in which a plurality of pieces of the corneal epithelium 51 are scattered onto a human amniotic membrane to have these pieces cultured into a laminated form (sheet) which is used in the corneal epithelium grafting operation.

In the case of the above-mentioned PRK operation, its advantages are enumerated as follows: namely, a first advantage resides in that the operation is very simple; a second advantage resides in that the operation is substantially free from any complication or disease concurrent with the incised wound, wherein, even when the disease appears concurrently with the incised wound, such disease is slight; and a third advantage resides in that healing of the incised wound is physiologically realized since the corneal epithelium 51 is high in activity in regeneration.

On the other hand, the following defects are enumerated: namely, a first defect resides in that a paint or a feeling of physical disorder continues for approximately 4 days or around until the corneal epithelium 51 is regenerated to cover the incised wound; a second defect resides in that the incised wound having been healed often (is apt to) results in dim (cloudy) sight in which a cloud appears under the corneal epithelium; and, a third defect resides in that the operation requires a long period use of a medicine for relief from the cloud of the cloudy sight appearing under the corneal epithelium.

Further, in the case of the above LASIK operation, its advantages are enumerated as follows: namely, a first advantage resides in that this operation is less painful than the PRK operation and is capable of realizing earlier recovery of the visual power of the patient than the PRK operation; a second advantage resides in that this operation is less suffering from the cloudy sight than the PRK operation in the aftercare treatment, wherein the cloud in the cloudy sight appears under the corneal epithelium; and, a third advantage resides in that this operation requires a less period use of a medicine for relief from the cloudy sight in which the cloud appears under the corneal epithelium.

On the other hand, defects of this operation are enumerated as follows: namely, a first defect resides in that this operation is more complicated than the PRK operation; a second defect resides in that this operation is more limited in applicability than the PRK operation, wherein an example in poor applicability of this operation is that the microkeratome is suitable for a straight incision use but not suitable for a curved incision use along a curvature of the cornea of the patient, wherein, since a width between an upper and a lower eyelid of an opened eye globe is narrow, it is impossible to set the suction ring; and, a third defect resides in that this operation more suffers from the disease concurrent with the incised wound both during the operation and after the operation than the PRK operation, wherein, once the concurrent disease appears, such concurrent disease tends to become a serious disease. As the concurrent disease, there are enumerated various types of abnormality, for example, in size, shape, location and in thickness together with perforation. A fourth defect of this operation resides in that the corneal stroma 55 is inferior in regeneration ability to the corneal epithelium 51, which results in a very slow healing of the incised wound and also in the non-physiological healing.

Next, in the case of the above-mentioned LASEK operation, there are enumerated the same advantages as those described in the PRK operation: namely, this operation is substantially free from the concurrent disease, wherein, even when the concurrent disease appears, such concurrent disease is light; this operation makes it possible for the incised wound to be physiologically healed since the regeneration ability of the corneal epithelium 51 is high; and, this operation is free from the same defect as that of the LASIK operation in which the interlayer incision of the corneal stroma 55 is made.

However, in the case of the LASEK operation, since the corneal epithelium sheet is subjected to the medication with alcohol, the cells of the epithelium are damaged to such as extent that all the cells are destroyed. Due to this, the LASEK operation is disadvantageous in that the healing of the incised wound of the corneal epithelium 51 is slow. In addition, the corneal epithelium sheet prepared through the LASEK operation is not constructed of all layers of the corneal epithelium 51. Due to this, the residues often remain on the exposed surface of the corneal stroma. This requires a removal operation of such residue from the surface of the corneal stroma. As described above, the LASEK operation is a delicate operation and requires much more operation time.

Next, in each of: the allograft operation; the grafting operation of a superficial layer of cornea; the grafting operation of a deep superficial layer of cornea; and, the corneal grafting operation in which culturing of the corneal epithelium sheet is conducted, the immune rejection of the corneal sheet occurs since the corneal sheet of another person is grafted to the patient. Further, in culturing the corneal sheet, a large-scaled facility or installation is required. In addition, since the corneal epithelium 51 is inherently constructed of a plurality of layers, a so-called high-technology is required in culturing such multi-layered corneal sheet. This requires much more culturing time.

### SUMMARY OF THE INVENTION

Consequently, it is an object of the present invention to provide a medical instrument used in a corneal surgical operation for preparing a complete corneal sheet in a short time and in an easy manner without performing any medication with a medicine which damages the corneal sheet, wherein: the corneal sheet is constructed exclusively of all the layers of the corneal epithelium only; and, the corneal sheet is prepared while enjoying the advantages inherent in the LASEK operation in the corneal refractive correction surgical operation.

Further, it is another object of the present invention to provide a medical instrument used in a corneal surgical operation for preparing a complete corneal sheet without using any culturing techniques and without suffering from any immune rejection which occurs in a corneal grafting operation conducted between a patient and another person, wherein the corneal sheet is constructed exclusively of all the layers of the corneal epithelium only.

In accordance with the first aspect of the present invention, the above objects of the present invention are accomplished by providing:

A medical instrument used in a corneal surgical operation comprising a member positioned in the boundary between a corneal epithelium and a corneal stroma, wherein the member is provided with: a resiliency which makes it possible for a first contact surface of the member to deform along a shape of a contact surface of the corneal stroma in response to a driving force applied to the member in a direction toward a bottom portion of the corneal stroma; and, a strength which makes it possible for a second contact surface of the member to push up the lowermost bottom surface or contact surface of the corneal epithelium when the the member is driven along the boundary surface between the corneal stroma and the corneal epithelium in response to a thrust applied in a direction along the boundary surface.

In this first aspect of the present invention, the member has: its first contact surface deform along the shape of the contact surface of the corneal stroma in response to the driving force applied to the member in a direction toward the bottom portion of the corneal stroma; and, a second contact surface push up the lowermost bottom surface or contact surface of the corneal epithelium when the member is driven along the boundary surface between the corneal stroma and the corneal epithelium in response to the thrust applied in a direction along the boundary surface.

In accordance with a second aspect of the pesent invention, the above objects of the present invention are accomplished by providing:

The medical instrument used in the corneal surgical operation as set forth in the first aspect, wherein the member is a thread-like member having a diameter of equal to or less than 100 µm. The member is constructed of a synthetic fiber or made of metal. Since a thickness of the corneal epithelium is approximately 50 µm, preferably the member has a diameter of from equal to or more than 30 µm to equal to or less than 50 µm.

In a third aspect of the present invention, the member is constructed of a synthetic fiber.

In a fourth aspect of the present invention: a supporting mechanism for supporting opposite ends of the member is further provided in the medical instrument; and, the supporting mechanism supports the opposite ends of the member in a condition in which a load is imposed on the member. Here, preferably the load is equal to or more than 1.9613 N. This load of equal to or more than 1.9613 N may realize a preferable strength of the member.

In a fifth aspect of the present invention, the driving force and the thrust are components of a force applied to the member at a predetermined angle which the second contact surface forms with a tangential line extending along a direction in which the thrust is applied.

In this aspect of the present invention, the driving force and the thrust are separately applied to the member. Instead thereof, a unidirectional force is applied to the member.

In a sixth aspect of the present invention, the predetermined angle is 45 degrees. This angle of 45 degrees is preferable when the load of equal to or more than 1.9613 is applied to the member.

In a seventh aspect of the present invention, a medical instrument used in a corneal surgical operation comprises:
a member positioned in the boundary between a corneal epithelium and a corneal stroma, wherein the member is provided with: a resiliency which makes it possible for a first contact surface of the member to deform along a shape of a contact surface of the corneal stroma in response to a driving force applied to the member in a direction toward a bottom portion of the corneal stroma; and, a strength which makes it possible for a second contact surface of the member to push up the lowermost bottom surface or contact surface of the corneal epithelium when the the member is driven along the boundary surface between the corneal stroma and the corneal epithelium in response to a thrust applied in a direction along the boundary surface;
a pair of supporting segment portions extending in parallel to each other, wherein the supporting segment portions support opposite ends of the member in a condition in which a load is imposed to the member;
a grip portion assuming a plate-like shape for applying both the driving, force and the thrust to the member; and
a connecting portion for connecting the grip portion with each of the supporting segment portions;
wherein a plane in which the grip portion lies and another plane in which the supporting segment portions lie are parallel to each other;
wherein the connecting portion extends in a direction forming a predetermined inclination angle with the plane in which the grip portion lies.

In this aspect of the present invention, even when the grip portion is gripped, since the connecting portion extends in a direction defined by an inclination angle, a concave position in which the eye globe is disposed is not disadvantageous in performing the above operation so that the corneal epithelium can be properly separated from the corneal stroma.

In an eighth aspect of the present invention, such predetermined inclination angle is 15 degrees. This inclination angle of 15 degrees makes it easy to set the predetermined angle at an angle of 45 degrees in view of an uneven outline or contour of a face of the patient when both the driving force and the thrust are components of a force, wherein: the force is applied to the member at a predetermined angle with respect to a tangential line extending along a direction in which the thrust is applied; and the second contact surface is driven on the contact surface of the corneal stroma.

In a ninth aspect of the present invention, a concave-convex unevenness is provided in at least one of surfaces of the above-mentioned grip portion. Due to the provision of such a concave-convex unevenness, the grip portion is capable of functioning as non-slip means when gripped in operation.

In a tenth aspect of the present invention, there is further provided a driving-force applying means for applying the above-mentioned driving force to the above-mentioned member.

In an eleventh aspect of the present invention, there is further provided a guide means for guiding the above-mentioned member when the member is moved in a thrust direction under the effect of the above-mentioned thrust.

In a twelfth aspect of the present invention, there is further provided a vibrating means for reciprocally moving or vibrating the above-mentioned member in a direction crossing the above-mentioned thrust direction.

In a thirteenth aspect of the present invention, there is further provided a thrust-applying means for applying the above-mentioned thrust to the above-mentioned member.

### BRIEF DESCRIPTION OF THE DRAWINGS

Fig. 1 is a front view of an instrument for preparing the corneal epithelium sheet, wherein the instrument serves as a medical instrument for carrying out a surgical operation of the cornea according to an embodiment of the present invention;
Fig. 2 is aplan view of the instrument for preparing the corneal epithelium sheet shown in Fig. 1;
Fig. 3 is a right side view of the instrument for preparing the corneal epithelium sheet shown in Fig. 1;
Fig. 4 is a bottom view of the instrument for preparing the corneal epithelium sheet shown in Fig. 1;
Fig. 5 is a sectional view, taken along the A-A line of Fig. 2;
Fig. 6 is an enlarged view of an essential portion, taken along the line B-B of Fig. 1;
Fig. 7 is a sectional view, taken along the line C-C of Fig. 6;
Fig. 8 is a sectional view, taken along the line D-D of Fig. 6;
Fig. 9 is a plan view of the cornea, illustrating a condition in which, after a horseshoe-shaped groove is formed in the cornea, the thread-like member 5 of the instrument 1 for preparing the corneal epithelium sheet is positioned in the boundary between the corneal epithelium and the corneal stroma;
Fig. 10 is a sectional view taken along the line E-E of Fig. 9, illustrating a condition in which the corneal epithelium is peeled from the corneal stroma;
Fig. 11 is a sectional view taken along the line F-F of Fig. 10, illustrating a condition in which the corneal epithelium is peeled from the corneal stroma;
Fig. 12 is an enlarged view of an essential portion of Fig. 10;
Fig. 13 is a view illustrating a condition in which the hinged corneal sheet is prepared, wherein the hinged corneal sheet is constructed exclusively of all the layers of the corneal epithelium only, which epithelium is peeled and turned over;
Fig. 14 is a front view of the instrument for preparing the corneal epithelium sheet, wherein the instrument serves as a medical instrument for carrying out the surgical operation of the cornea according to another embodiment of the present invention;
Fig. 15 a sectional view, taken along the line G-G of Fig. 14;
Fig. 16 is a schematic block diagram of a medical instrument for carrying out the surgical operation of the cornea according to still another embodiment of the present invention;
Fig. 17 is a view showing another example of the thrust direction in which the thread-like member is driven;
Fig. 18 is a view illustrating a plurality of the thread-like members employed in the example shown in Fig. 17;
Fig. 19 is a sectional view showing the cornea in construction; and
Fig. 20 is a view illustrating a corneal refractive correction operation called "LASEK", in which the impregnation of the cornea with a medicine is carried out by pouring alcohol into the alcohol receiver disposed on the cornea, through which impregnation the hinged corneal epithelium sheet become free from the corneal stroma shown here.

### DETAILED DESCRIPTION OF THE PREFERRED EMBODIMENTS

The best modes for carrying out the present invention will be described using preferred embodiments of the present invention with reference to the accompanying drawings.

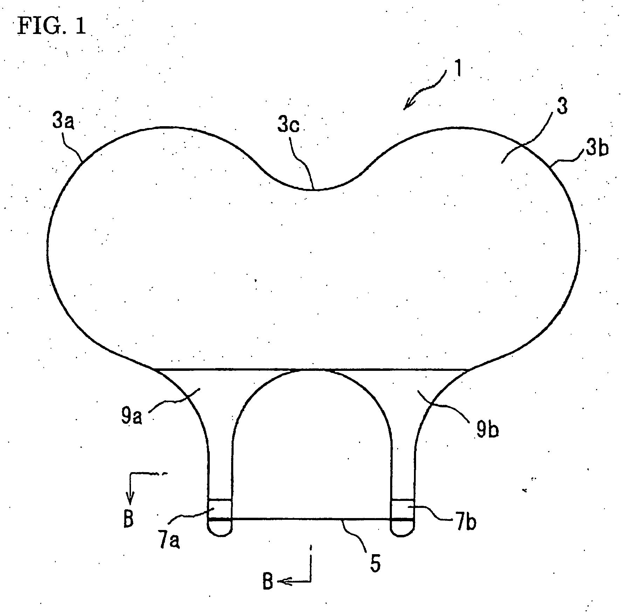

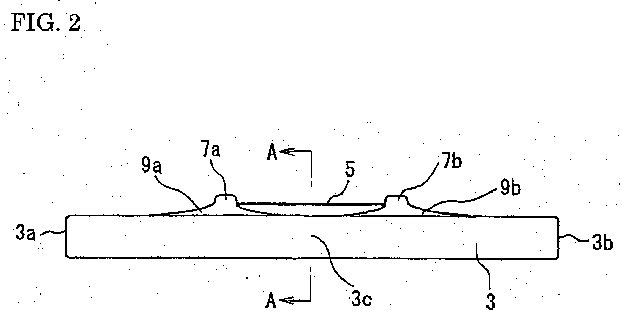

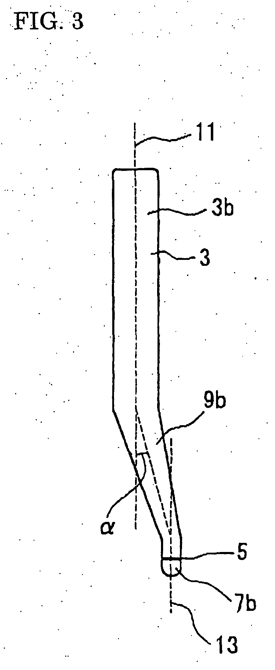

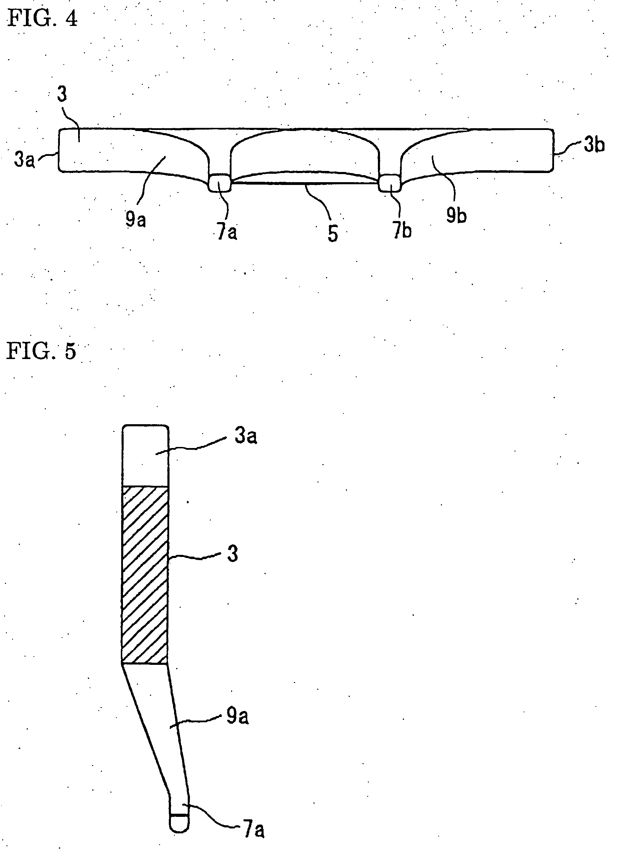

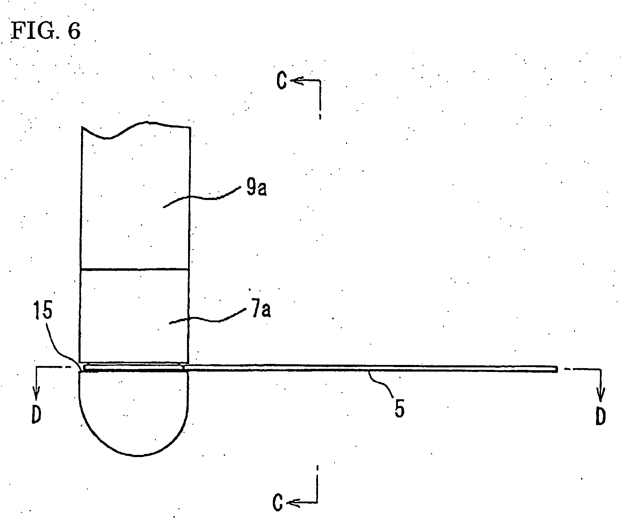

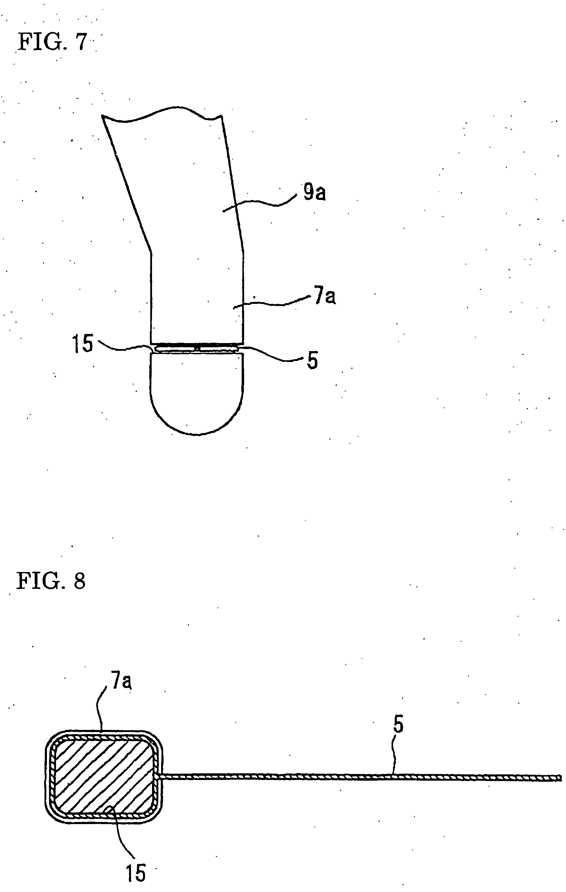

Fig. 1 is a front view of an instrument for preparing the corneal epithelium sheet, wherein the instrument serves as a medical instrument for carrying out a surgical operation of the cornea according to an embodiment of the present invention. Fig. 2 is a plan view of the instrument for preparing the corneal epithelium sheet shown in Fig. 1. Fig. 3 is a right side view of the instrument for preparing the corneal epithelium sheet shown in Fig. 1. Fig. 4 is a bottom view of the instrument for preparing the corneal epithelium sheet shown in Fig. 1. Fig. 5 is a sectional view, taken along the A-A line of Fig. 2. Fig. 6 is an enlarged view of an essential portion, taken along the line B-B of Fig. 1. Fig. 7 is a sectional view, taken along the line C-C of Fig. 6. Fig. 8 is a sectional view, taken along the line D-D of Fig. 6.

The instrument 1 for preparing the corneal epithelium sheet is provided with: a grip portion 3 having a thickness of 5 mm and assuming a flat plate-like shape; a thread-like member 5 constructed of a synthetic fiber having a diameter of equal to or less than 10 µm; a pair of supporting segment portions 7a, 7b each for supporting each of opposite end portions of the thread-like member 5, wherein the supporting segment portions 7a, 7b are disposed in parallel to each other; connecting portions 9a, 9b for connecting the grip portion 3 and each of the supporting segment portions 7a, 7b.

An outer peripheral shape of the grip portion 3 is straight in its connecting area through which the grip portion 3 is connected with each of the The thread-like member 5. As for the remaining area of the outer peripheral shape of the grip portion 3, the grip portion 3 has opposite circularly-curved portions 3a, 3b, disposed between which is a concave portion 3c. These portions 3a, 3b, 3c are connected with each other in shape. The thread-like member 5 is smooth in surface. An annular groove 15 is formed in each of the supporting segment portions 7a, 7b. The thread-like member 5 has its opposite ends fixed to the annular groove 15 and supported by the groove 15. Applied to this thread-like member 5 is a load of equal to or more than 1.9613 N (200 g weight). The connecting portions 9a, 9b are provided in a manner such that: a plane 11 in which the grip portion 3 lies is parallel to another plane 13 in which both the supporting segment portions 7a, 7b lie; and, each of the connecting portions 9a, 9b forms an inclination angle "α" of 15 degrees with each of these planes 11, 13.

Hereinbelow, the corneal refractive correction surgical operation carried out by using the instrument 1 for preparing the corneal epithelium sheet will be described.

First of all, eyedropper-type anesthesia of the cornea is conducted and an eye opener is used to open eyelids. Then, a horseshoe-shaped groove-like separation or peel of the corneal epithelium 51 is conducted. The horseshoe-shaped groove has a depth of from at least 40 µm to up to 50 µm, and has a width of from at least 0.5 mm to up to 1.0 mm.

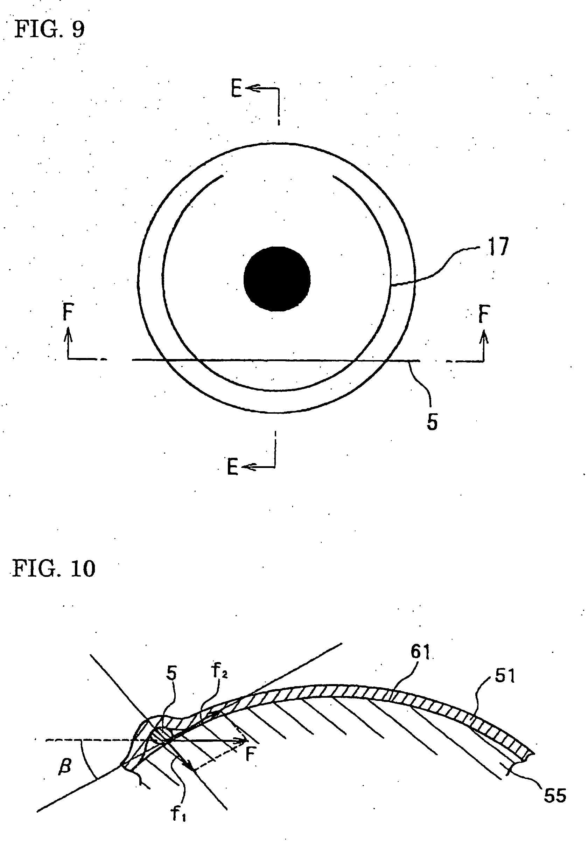

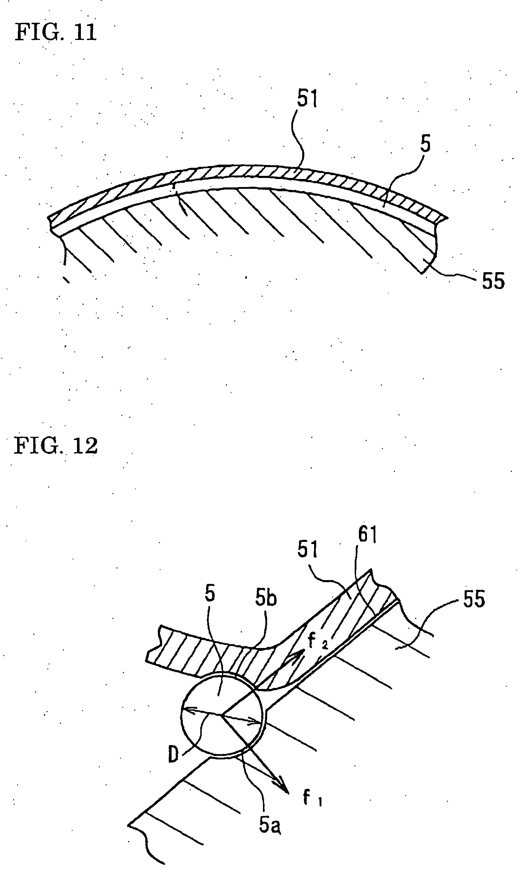

Fig. 9 is a plan view of the cornea, illustrating a condition in which, after a horseshoe-shaped groove is formed in the cornea, the thread-like member 5 of the instrument 1 for preparing the corneal epithelium sheet is positioned in the boundary between the corneal epithelium and the corneal stroma. Fig. 10 is a sectional view taken along the line E-E of Fig. 9, illustrating a condition in which the corneal epithelium is peeled from the corneal stroma. Fig. 11 is a sectional view taken along the line F-F of Fig. 10, illustrating a condition in which the corneal epithelium peeled from the corneal stroma. Fig. 12 is an enlarged view of an essential portion of Fig. 10. The corneal epithelium is peeled from the corneal stroma in the following manner:

A surgical operator applies a force F to the thread-like member 5 when the member 5 is reciprocally moved or vibrated in its extending direction with an amplitude of approximately 10 mm of its vibration. The force F is divided into: a component force f1 serving as a driving force directed toward the bottom side of the corneal stroma 55; and, another component force f2 serving as a thrust directed parallel to a boundary surface 61 between the corneal epithelium 51 and the corneal stroma 55. The thread-like member 5 has a resiliency to make it possible for a first contact surface 5a of the thread-like member 5 to be deformed along the contour of a contact surface of the corneal stroma 55 in response to the driving force thus applied to the thread-like member 5. At this time, the thread-like member 5 has a strength to make it possible for a second contact surface 5b of the thread-like member 5 to push up the lowermost contact surface of the corneal epithelium 51 and move along the boundary surface 61.

The direction in which the force F is exerted on the thread-like member 5 forms an angle β of 45 degrees with a tangential line extending along a direction in which the second contact surface 5b of the thread-like member 5 is moved under the influence of the above-mentioned thrust exerted at the contact surface of the corneal stroma 55.

On the other hand, the first contact surface 5a and the second contact surface 5b are brought into area contact with the corneal stroma 55 and the corneal epithlium 51, respectively. Under such circumstances, the thread-like member 5 receives the force F in a manner such that the thread-like member 5 never cuts any one of the corneal epithelium 51 and the corneal stroma 55.

Incidentally, although the thread-like member 5 is described to have a diameter of equal to or less than 100 µm, preferably the thread-like member 5 has a diameter of equal to or less than 50 µm since the corneal epithelium 51 has a thickness of approximately 50 µm. As for the load, although a preferably value of the load is equal to or more than 0.98065 N (100 gram weight), in order to make it. possible for the thread-like member 5 to bear a load of 1.9613 N (200 gram weight), preferably the thread-like member 5 has a diameter D of equal to or more than 30 µm.

Further, it is also possible to have the thread-like member 5 constructed of a synthetic fiber and the like, or made of metals such as tungsten and the like.

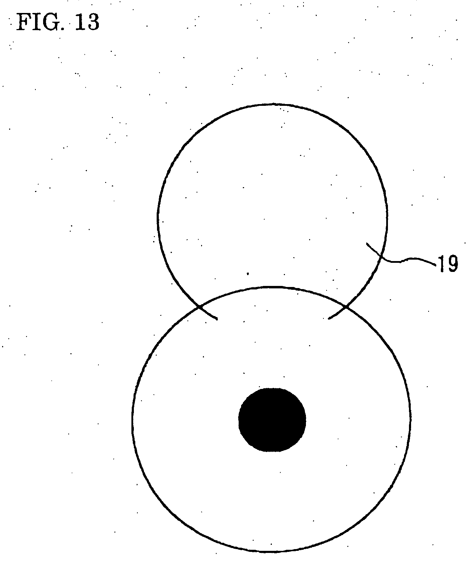

Fig. 13 is a view illustrating a condition in which the hinged corneal sheet is prepared, wherein the hinged corneal sheet is constructed exclusively of all the layers of the corneal epithelium only, which epithelium is then peeled and turned over.

As described above, the surgical operator separates the corneal epithelium 51 from the corneal stroma 55 to prepare the corneal epithelium 19, which is constructed exclusively of the corneal. epithelium 51 only. Then, in order to expose a surface of the corneal stroma 55, the corneal epithelium sheet 19 is peel and turned over.

After that, when the residues of the corneal epithelium 51 are found, such residues are removed. On the other hand, the uppermost surface of the thus exposed corneal stroma 55 is exposed to radiation of an excimer laser, so that the surface layer of the corneal stroma 55 is ablated by the laser, whereby the corneal stroma is modified in shape, which results in a change of the cornea in its refractive force. After that change, the corneal epithelium 19 is replaced or returned to its initial position. At this time, the corneal epithelium sheet 19 is free from any damage such as ones caused by medication. Due to this, all the cells of the epithelium are kept alive. In the post-surgical operative aftercare, for example, therapeutic-type contact lenses are used in wearing.

It is possible for the instrument 1 for preparing the corneal epithelium sheet to prepare a complete cornea sheet easily in a short time, without employing any medication damaging the cornea, while enjoying all the advantages of the conventional LASEK, wherein: the complete cornea sheet is constructed exclusively of all the layers of the corneal epithelium only; and, the instrument 1 for preparing the corneal epithelium sheet makes it possible to perform the above-mentioned "corneal refractive correction surgical operation". In addition, by using such instrument 1 which is for preparing the corneal epithelium sheet and makes it possible to perform the above-mentioned "corneal refractive correction surgical operation", it is possible to safely prepare such a complete cornea sheet without damaging the corneal stroma 55, wherein the complete cornea sheet is constructed exclusively of all the layers of the corneal epithelium only. As a result, in effect, a regeneration process of the corneal epithelium 51, which process comprises the steps of: extending the corneal epithelium 51; contacting the corneal epithelium 51; and, increasing the corneal epithelium 51 in thickness, is accomplished in a sound state of the outermost surface of the corneal stroma 55 in the physiologically shortest possible period of time.

Further, the instrument 1 for preparing the corneal epithelium sheet can be used also in the corneal epithelium grafting surgical operation. In this case, the following advantages are obtained: by using the instrument 1 for preparing the corneal epithelium sheet, it is possible for the surgical operator to prepare a non-hinged sheet of a sound eye globe of a patient, wherein the non-hinged sheet is constructed exclusively of all the layers of the corneal epithelium of the eye globe only and is separated from the eye globe; then, the surgical operator grafts this corneal epithelium sheet to the other eye globe of the same patient. Due to this, there is no fear of any immune rejection, which usually occurs between a patient and a donor. Further, as for the corneal epithelium sheet to be grafted, since there is no need for culturing of the sheet, there is no need for any large-scaled installation and any extended period of time for culturing the sheet. In addition, it is possible to timely perform the epithelium grafting surgical operation with respect to a patient requiring such operation. In addition, since the sheet is constructed exclusively of all the layers of the corneal epithelium's cells only, which cells are active in cytodieresis to replace old cells with new ones, and further since a surface of the stroma itself from which the epithelium sheet is separated is free from any damage, an incised wound of the sound-side one of the eye globes of the patient from which sound-side one the epithelium sheet is separated for grafting purpose is curable in the physiologically shortest possible period of time due to the corneal epithelium's cells being active in cytodieresis. Further, an incised wound of the other of the eye globe of the patient is free from any immune rejection, and therefore curable in the physiologically shortest possible period of time immediately after completion of the grafting operation since the cells of the corneal epithelium are active in cytodieresis.

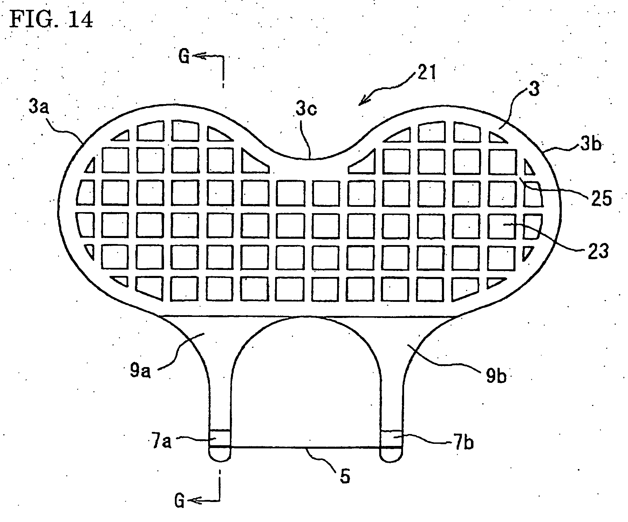

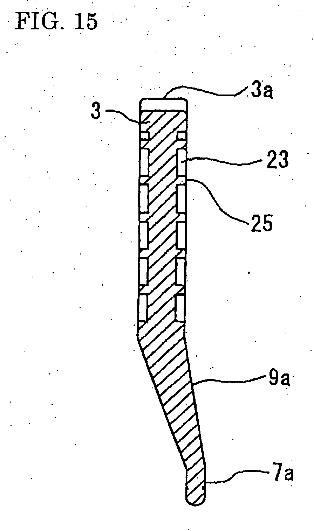

Fig. 14 is a front view of the instrument for preparing the corneal epithelium sheet, wherein the instrument serves as a medical instrument for carrying out the surgical operation of the cornea according to another embodiment of the present invention. Fig. 15 is a sectional view, taken along the line G-G of Fig. 14.

In opposite surface of a grip portion 3 of an instrument 21 for preparing the corneal epithelium sheet, there are arranged a plurality of grid-like concave portions 23 defined by a plurality of crossed convex portions 25.

Due to the provision of both such concave portions 21 and convex portions 25, the surgical operator is substantially prevented from having his or her hands slipped on the instrument 21. This enables the surgical operator to properly use the instrument 21 for preparing the corneal epithelium sheet.

Incidentally, the above-mentioned concave and convex portions formed in the grip portion 3 is not necessarily formed in both the opposite surfaces of the grip portion 3. It is also possible to form these concave and convex portions only in one of the opposite surfaces of the grip portion 3. Further, in cross section, the concave and convex portions may assume various type of shapes, for example such as a wave-like shape in groove or an elongated-mountain-like shape.

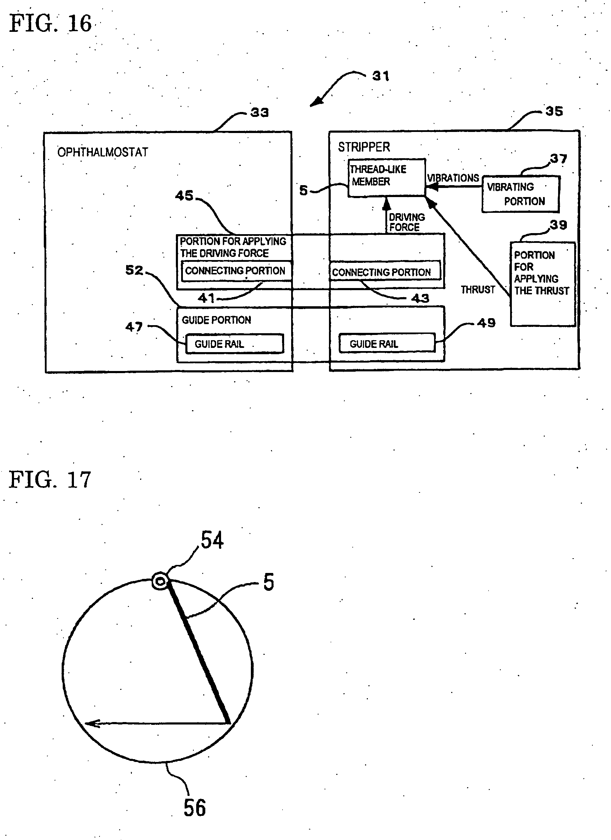

Fig. 16 is a schematic block diagram of a medical instrument for carrying out the surgical operation of the cornea according to still another embodiment of the present invention.

Amedical instrument 31 for carrying out the surgical operation of the cornea is provided with: an eye-globe fixing apparatus or ophthalmostat 33; and, a stripper 35. For example, in concrete form, the ophthalmostat 33 is constructed of a suction ring. On the other hand, in concrete form, for example, the stripper 35 is constructed of an improved one of micro-keratomes.

The stripper 35 is provided with: the thread-like member 5; a vibrating portion 37 for reciprocally moving or vibrating the thread-like member 5 in its extending direction with an amplitude of approximately 10 mm in its vibration; and, aportion 39 for applying a thrust in a direction crossing a vibrating direction of the thread-like member 5.

Further, the ophthalmostat 33 is provided with a connecting portion 41. On the other hand, the stripper is provided with another connecting portion 43. Both the ophthalmostat 33 and the stripper 35 are designed in dimension in a manner such that: when both the connecting portions 41, 43 are connected with each other, a driving force is applied to the bottom side of the corneal stroma 55 through the thread-like member 5. In other words, a portion 45 for applying the driving force to the thread-like member 5 is constructed of: the connecting portion 41 of the ophthalmostat 33; and, the connecting portion 43 of the stripper 35.

The ophthalmostat 33 is provided with a guide rail 47. On the other hand, the stripper 35 is provided with another guide rail 49. A direction, in which the thread-like member 5 is driven, is determined or established when both the guide rails 47, 49 mesh together. In other words, a guide portion 52 for guiding the thread-like member 5 in operation when the member 5 is driven by a thrust applied by the portion 39 far applying the thrust is constructed of: the guide rail 47 of the ophthalmostat 33; and, the guide rail 49 of the stripper 35.

Automation of the surgical operation is realized using such instruments to make it possible to prepare the corneal sheet, which is constructed exclusively of all the layers of the corneal epithelium 51. Incidentally, in the embodiment shown in Fig. 16, through the ophthalmostat 33 and the stripper 35 are separately formed from each other, it is also possible to integrate the ophthalmostat 33 and the stripper 35 into one system.

Fig. 17 is a view showing another example of the thrust direction in which the thread-like member is driven.

In the embodiments described in the above, the direction in which the thread-like member 5 is driven crosses the extending direction of the thread-like member 5, for example such as a direction perpendicular to the direction in which thread-like member is driven, so that the entire thread-like member 5 is driven. However, as shown in Fig. 17, it is also possible to drive the thread-like member 5 in a different manner such that: one of opposite ends of the thread-like member 5 is fixed to form a fixed end portion 54; and, the other of the opposite ends of the thread-like member 5 is moved in a direction indicated by the arrow.

Incidentally, it is also possible to provide a spring in the other end of the thread-like member 5 in a manner such that: a load of equal to or more than 1.9613 N (200 gram weight) is applied to the thread-like member 5 in a condition in which the other end of the thread-like member 5 travels along a circular arc 56.

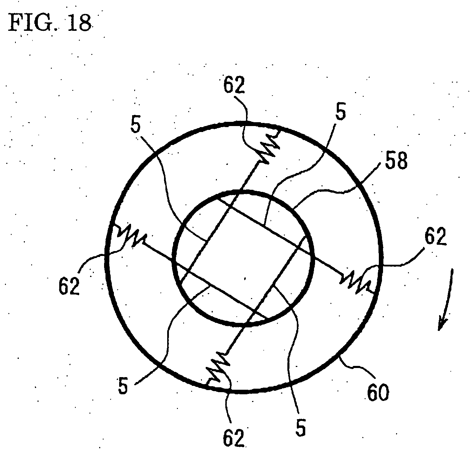

Fig. 18 is a view illustrating a plurality of the thread-like members employed in the example shown in Fig. 17.

One of the opposite ends of each of four pieces of the thread-like members 5 is fixed onto a sleeve-like stationary circle 58, wherein these four pieces of the thread-like members 5 have their fixed ends described above fixed at equal intervals onto the stationary circle 58. Under such circumstances, a spring 62 for applying a load of equal to or more than 1.9613 N (200 gram weight) to the thread-like member 5 is provided in each of the remaining ends of these four thread-like members 5. Then, four pieces of springs 62 are mounted on a rotating circle 60 at equal intervals. In this embodiment employing a plurality of the thread-like members 5, in operation, the stationary circle 58 is kept stationarily when the rotating circle 60 is rotated in the direction indicated by the arrow.

Incidentally, in the embodiments described above, as a member for separating the corneal epithelium 51 from the corneal stroma 55, there is employed the thread-like member 5. However, it is also possible to use a plate-like member in place of the thread-like member 5. When the plate-like member is used, the plate-like member is provided with: a first contact surface in one of opposite surfaces of the plate-like member, wherein the first contact surface varies in shape in response to variations in shape of the contact surface of the corneal stroma 55 which varies in response to the driving force applied in the direction toward the bottom surface of the corneal stroma 55; and, a second contact surface in the other of the opposite surfaces of the plate-like member, wherein the second contact surface is driven along the boundary surface 61 in a condition in which the second contact surface pushes upward the contact surface or lowermost surface of the corneal epithelium 51 in response to the thrust applied along the boundary surface between the corneal epithelium 51 and the corneal stroma 55.

Further, in the embodiments described in the above, the resultant or sum of the driving force and the thrust with respect to the thread-like member 5 becomes the force F, wherein the force F is applied at a predetermined angle of 45 degrees with respect to the tangential line extending along the direction in which the second contact surface 5b of the thread-like member 5 is driven in the contact surface of the corneal stroma 55. Consequently, in each case of the instruments 1, 21 each for preparing the corneal epithelium sheet, it is also possible to provide a means for determining the above-mentioned predetermined angle together with an indication means for indicating such predetermined angle. Further, in the case of the medical instrument 31 for carrying out the surgical operation of the cornea shown in Fig. 16, it is also possible to provide the means for determining the predetermined angle, which means issues results in measurement of the predetermined angle, wherein such results are used in controlling the predetermined angle by using a control means which is also provided in the medical instrument 31.

Further, the present invention is not limited to the above embodiments. So, it is needless to say that the present invention may be modified into various forms within the scope of technical idea of the present invention defined in the following claims.

### Re: Industrial Applicability:

According to the present invention, it becomes possible to separate the corneal epithelium from the corneal stroma along the boundary between the corneal epithelium and the corneal stroma without cutting any one of the corneal epithelium and the corneal stroma even when the member having been positioned in the boundary between the corneal epithelium from the corneal stroma is driven by the driving force and the thrust, because: the first contact surface and the second contact surface of the above-mentioned member are brought into area-contact with the corneal stroma and the corneal epithelium, respectively.

Due to this, in the corneal refractive correction surgical operation using the medical instrument of the present invention for carrying out the surgical operation of the cornea, it is possible to prepare safely the complete cornea sheet easily in a short time without carrying out medication which will damage the cornea, wherein the complete cornea sheet is constructed exclusively of all the layers of the corneal epithelium only. Further, in this corneal refractive correction surgical operation using the medical instrument of the present invention for carrying out the surgical operation of the cornea, since the corneal epithelium is constructed exclusively of all the layers of the corneal epithelium only, there is no fear of any disease concurrent with the separation of the corneal epitherlium from the corneal stroma. Even if such disease appears concurrently with the separation of the corneal epitherlium from the corneal stroma, such disease will be alleviated. Further, since the corneal epithelium is active in regeneration ability thereof to facilitate the physiological cure of the incised wound of the cornea, the present invention is substantially free from any possible defect which will be caused by an interlayer incision. of the corneal stroma itself. The above advantages are inherent in the present invention.

Still further, in the corneal refractive correction surgical operation using the medical instrument of the present invention for carrying out the surgical operation of the cornea, it is possible to prepare the corneal sheet from a sound one of the eye globes of the patient himself or herself, wherein the corneal sheet is constructed exclusively of all the layers of the corneal epithelium of the sound eye globe. Due to this, in the present invention, there is no fear of immune rejection against the grafted tissue, which rejection is usually observed when such grafting operation is conducted between the patient and a donor different from the patient himself or herself. Further, in the present invention, there is no need for culturing the corneal sheet for conducting the grafting operation thereof. Consequently, there is also no need for a large-scaled facility for conduction the culture of the corneal sheet. In addition, there is also no need for high-technologies required in reproducing the corneal sheet through its culturing process. This makes it possible to save time since any culturing period of time is not required in the present invention. As a result, in the present invention, it is possible to prepare the corneal sheet in a short time in an easy manner at any time even in an urgent case or operation, wherein the corneal sheet is constructed exclusively of all the layers of the corneal epithelium only. The above advantages are inherent in the present invention. In addition, the corneal sheet of the present invention is constructed exclusively of all the layers of the corneal epithelium, cells of which is particularly active in cytodieresis to facilitate replacement of the old cells with new ones in the corneal epithelium. Further, a surface of the corneal stroma, which surface remains in the eye globe after completion of the incision of the corneal epithelium thereon, is free from any damage. Due to this, the incised wound of the sound one of eye globes of the patient having been subjected to the incision and separation of the corneal epithelium sheet for the grafting operation thereof is capable of being cured in the physiologically shortest possible time due to the active cytodieresis of the cells of the corneal epithelium. Further, another incised wound appearing in the other (which receives the corneal sheet) of eye globes of the patient is free from any immune rej ection, and therefore capable of enjoying a physiological quick start of curing of the incised wound immediately after completion of the grafting operation of the corneal sheet, wherein such quick start of curing of the incised wound is realized by the active cytodieresis of the corneal epithelium's cells. The above advantages are inherent in the present invention.

## Claims

1. A medical instrument used in a corneal surgical operation comprising a member positioned in the boundary between a corneal epithelium and a corneal stroma, wherein said member is provided with: a resiliency which makes it possible for a first contact surface of said member to deform along a shape of a contact surface of said corneal stroma in response to a driving force applied to said member in a direction toward a bottom portion of said corneal stroma; and, a strength which makes it possible for a second contact surface of said member to push up the lowermost bottom surface or contact surface of said corneal epithelium when the said member is driven along the boundary surface between said corneal stroma and said corneal epithelium in response to a thrust applied in a direction along the boundary surface.

2. The medical instrument used in the corneal surgical operation as set forth in claim 1, wherein said member is a thread-like member having a diameter of equal to or less than 100 µm.

3. The medical instrument used in the corneal surgical operation as set forth in claim 1, wherein said member has a diameter of from equal to or more than 30 µm to equal to or less than 50 µm.

4. The medical instrument used in the corneal surgical operation as set forth in claim 2 or 3, wherein said member is constructed of a synthetic fiber.

5. The medical instrument used in the corneal surgical operation as set forth in any one of claims 1, 2 and 3, wherein: a supporting mechanism for supporting opposite ends of said member is further provided in the medical instrument; and, said supporting mechanism supports said opposite ends of said member in a condition in which a load is imposed on said member.

6. The medical instrument used in the corneal surgical operation as set forth in claim 5, wherein said load is equal to or more than 1.9613 N.

7. The medical instrument used in the corneal surgical operation as set forth in claim 1, wherein: said driving force and said thrust are components of a force applied to said member at a predetermined angle which said second contact surface forms with a tangential line extending along a direction in which said thrust is applied.

8. The medical instrument used in the corneal surgical operation as set forth in claim 7, wherein said predetermined angle is 45 degrees.

9. A medical instrument used in a corneal surgical operation comprising:
a member positioned in the boundary between a corneal epithelium and a corneal stroma, wherein said member is provided with: a resiliency which makes it possible for a first contact surface of said member to deform along a shape of a contact surface of said corneal stroma in response to a driving force applied to said member in a direction toward a bottom portion of said corneal stroma; and, a strength which makes it possible for a second contact surface of said member to push up the lowermost bottom surface or contact surface of said corneal epithelium when the said member is driven along the boundary surface between said corneal stroma and said corneal epithelium in response to a thrust applied in a direction along the boundary surface;
a pair of supporting segment portions extending in parallel to each other, wherein said supporting segment portions support opposite ends of said member in a condition in which a load is imposed to said member;
a grip portion assuming a plate-like shape for applying both said driving force and said thrust to said member; and
a connecting portion for connecting said grip portion with each of said supporting segment portions;
wherein a plane in which said grip portion lies and another plane in which said supporting segment portions lie are parallel to each other;
wherein said connecting portion extends in a direction forming a predetermined inclination angle with said plane in which said grip portion lies.

10. The medical instrument used in the corneal surgical operation as set forth in claim 9, wherein said predetermined inclination angle is 15 degrees.

11. The medical instrument used in the corneal surgical operation as set forth in claim 9 or 10, wherein a concave-convex unevenness is formed in at least one of opposite surfaces of said grip portion.

12. The medical instrument used in the corneal surgical operation as set forth in claim 9, wherein said load is equal to or more than 1.9613 N.

13. The medical instrument used in the corneal surgical operation as set forth in claim 9 or 12, wherein said member is a thread-like member having a diameter of equal to or less than 100 µm.

14. The medical instrument used in the corneal surgical operation as set forth in claim 13, wherein said member is constructed of a synthetic fiber.

15. The medical instrument used in the corneal surgical operation as set forth in claim 9 or 12, wherein said member has a diameter of from equal to or more than 30 µm to equal to or less than 50 µm.

16. The medical instrument used in the corneal surgical operation as set forth in claim 15, wherein said member is constructed of a synthetic fiber.

17. A medical instrument used in a corneal surgical operation comprising:
amember positioned in the boundarybetween a corneal epithelium and a corneal stroma, wherein said member is provided with: a resiliency which makes it possible for a first contact surface of said member to deform along a shape of a contact surface of said corneal stroma in response to a driving force applied to said member in a direction toward a bottom portion of said corneal stroma; and, a strength which makes it possible for a second contact surface of said member to push up the lowermost bottom surface or contact surface of said corneal epithelium when the said member is driven along the boundary surface between said corneal stroma and said corneal epithelium in response to a thrust applied in a direction along the boundary surface; and
a means for applying a driving force to said member.

18. The medical instrument used in the corneal surgical operation as set forth in any one of claims 17, wherein: a guide means for guiding sad member when said member is driven by said thrust is further provided in the medical instrument.

19. The medical instrument used in the corneal surgical operation as set forth in any one of claims 17 or 18, wherein: a vibrating means for reciprocally moving or vibrating said member in a direction crossing a direction in which said member is driven by said thrust is further provided in the medical instrument.

20. The medical instrument used in the corneal surgical operation as set forth in any one of claims 19, wherein: a means for applying said thrust to said member is further provided in the medical instrument.

21. A medical instrument used in a corneal surgical operation comprising:
a member positioned in the boundary between a corneal epithelium and a corneal stroma, wherein said member is provided with: a resiliency which makes it possible for a first contact surface of said member to deform along a shape of a contact surface of said corneal stroma in response to a driving force applied to said member in a direction toward a bottom portion of said corneal stroma; and, a strength which makes it possible for a second contact surface of said member to push up the lowermost bottom surface or contact surface of said corneal epithelium when the said member is driven along the boundary surface between said corneal stroma and said corneal epithelium in response to a thrust applied in a direction along the boundary surface; and
a guide means for guiding said member when said member is driven by said thrust.

22. A medical instrument used in a corneal surgical operation comprising:
a member positioned in the boundary between a corneal epithelium and a corneal stroma, wherein said member is provided with: a resiliency which makes it possible for a first contact surface of said member to deform along a shape of a contact surface of said corneal stroma in response to a driving force applied to said member in a direction toward a bottom portion of said corneal stroma; and, a strength which makes it possible for a second contact surface of said member to push up the lowermost bottom surface or contact surface of said corneal epithelium when the said member is driven along the boundary surface between said corneal stroma and said corneal epithelium in response to a thrust applied in a direction along the boundary surface; and
a vibrating means for reciprocally moving or vibrating said member in a direction crossing a direction in which said member is driven by said thrust.

23. A medical instrument used in a corneal surgical operation comprising:
a member positioned in the boundary between a corneal epithelium and a corneal stroma, wherein said member is provided with: a resiliency which makes it possible for a first contact surface of said member to deform along a shape of a contact surface of said corneal stroma in response to a driving force applied to said member in a direction toward a bottom portion of said corneal stroma; and, a strength which makes it possible for a second contact surface of said member to push up the lowermost bottom surface or contact surface of said corneal epithelium when the said member is driven along the boundary surface between said corneal stroma and said corneal epithelium in response to a thrust applied in a direction along the boundary surface; and
a means for applying said thrust to said member.
